# DEMANDE DE BREVET EUROPEEN

(11) **EP 2 098 183 A1**
(43) Date de publication de la demande: **09.09.2009**
(21) Numéro de dépôt: 08001710.6
(22) Date de dépôt: 30.01.2008
(51) Int. Cl.: A61B 17/70

(54) **Implant inter-épineux destiné à être implanté entre les apophyses épineuses de deux vertrèbres adjacentes**

(71) Demandeur: ARCA MEDICA GmbH, 7935 Neuenburg am Rhein (DE)
(72) Inventeur: Laurent, Benoît, 68100 Mulhouse (FR); Linge, Armand, 4457 Diegten (CH)
(74) Mandataire: Maillet, Alain

(57) **Abrégé**

La présente invention concerne un implant inter-épineux (1) destiné à être implanté entre les apophyses épineuses de deux vertèbres adjacentes de la colonne vertébrale, qui puisse être ancré de manière efficace et durable aux apophyses épineuses sans provoquer la fragilisation de celles-ci, qui limite les mouvements de flexion et d'extension et qui empêche les mouvements de rotation.
A cet effet, un implant inter-épineux selon l'invention, destiné â être implanté entre deux vertèbres adjacentes, est de type monobloc et comprend un corps central (11) sensiblement en forme de U comportant deux branches (10,11), et deux moyens d'ancrage (12,13) aux apophyses épineuses desdites vertèbres faisant saillie de la face extérieure (10a,11a) des deux branches dudit corps central, respectivement, caractérisé en ce que lesdits moyens d'ancrage sont des moyens d'enserrage desdites apophyses épineuses, chaque moyen d'enserrage (12) étant composé de deux demi-anses (121,122) comportant chacune une extrémité libre courbée (1212) en direction de l'extrémité libre (1222) de l'autre demi-anse (122) et présentant chacune une longueur suffisante pour que, lorsque ledit implant est implanté entre lesdites vertèbres, lesdites demi-anses se rejoignent dans une position de fermeture dudit moyen d'enserrage autour de ladite apophyse épineuse dans laquelle l'extrémité libre courbée d'une demi-anse chevauche l'extrémité libre courbée de l'autre demi-anse, ledit implant comportant, de plus, un moyen de blocage et de réglage (15) de la position de fermeture desdites extrémités libres courbées se chevauchant.
L'implant inter-épineux selon l'invention possède des moyens d'ancrage aux apophyses épineuses particulièrement avantageux.

## Description

La présente invention concerne un implant inter-épineux destiné à être implanté entre les apophyses épineuses de deux vertèbres adjacentes, par exemple, dans le cadre d'une sténose canalaire ou d'une discopathie.

La colonne vertébrale, ou le rachis, est composée de vertèbres regroupées en cinq régions que sont le rachis cervical, le rachis dorsal, le rachis lombaire, le rachis sacré et le rachis coccygien.

Un certain nombre de pathologies de la colonne vertébrale peuvent affecter les mouvements d'un patient et provoquer des douleurs parfois handicapantes. De telles pathologies sont par exemple, un syndrome facettaire articulaire, une sténose canalaire lombaire ou un disque intervertébral défectueux. Une des solutions proposées aujourd'hui pour traiter un certain nombre de ces pathologies est le recours à la voie chirurgicale et la mise en place d'un implant dans la zone du rachis à soulager.

Un certain nombre d'implants intervertébraux a été développé depuis de nombreuses années. Les implants sont généralement constitués de deux sortes d'éléments, des éléments occupant l'espace intersomatique et des éléments pourvus de moyens d'ancrage osseux, par exemple, d'ancrage aux apophyses épineuses des vertèbres.

On connaît, par exemple, un implant constitué d'un corps central en forme de U destiné à être inséré entre les apophyses épineuses de deux vertèbres consécutives et pourvu de deux paires d'oreilles faisant saillie de part et d'autre du corps central et délimitant chacune un évidement dans lequel vient se loger l'apophyse épineuse de chaque vertèbre. L'implant est alors fixé aux apophyses épineuses par pincement des oreilles. La fixation peut être renforcée par l'intermédiaire de moyens de fixation constitués de vis qui sont vissées dans l'os. Un tel moyen d'ancrage aux apophyses épineuses provoque, à terme, un descellement de l'implant. En effet, suite à la répétition des mouvements du patient dans le plan sagittal, c'est-à-dire de flexion et d'extension, l'espace occupé par les vis dans l'os s'élargit ce qui a pour conséquence que la fixation de l'implant aux apophyses épineuses n'est plus efficace ni sûre. Dans un tel cas, les mouvements de flexion et d'extension du patient ne sont plus limités et, de plus, les apophyses épineuses des vertèbres concernées se trouvent abîmées et donc fragilisées.

Le but de la présente invention est de proposer un implant inter-épineux destiné à être implanté entre les apophyses épineuses de deux vertèbres adjacentes de la colonne vertébrale, qui puisse être ancré de manière efficace et durable aux apophyses épineuses sans provoquer la fragilisation de celles-ci, qui limite les mouvements de flexion et d'extension et qui empêche les mouvements de rotation.

A cet effet, un implant inter-épincux selon l'invention, destiné à être implanté entre deux vertèbres adjacentes, est de type monobloc et comprend un corps central sensiblement en forme de U comportant deux branches, et deux moyens d'ancrage aux apophyses épineuses desdites vertèbres faisant saillie de la face extérieure des deux branches dudit corps central, respectivement, caractérisé en ce que lesdits moyens d'ancrage sont des moyens d'enserrage desdites apophyses épineuses, chaque moyen d'enserrage étant composé de deux demi-anses comportant chacune une extrémité libre courbée en direction de l'extrémité libre de l'autre demi-anse et présentant chacune une longueur suffisante pour que, lorsque ledit implant est implanté entre lesdites vertèbres, lesdites demi-anses se rejoignent dans une position de fermeture dudit moyen d'enserrage autour de ladite apophyse épineuse dans laquelle l'extrémité libre courbée d'une demi-anse chevauche l'extrémité libre courbée de l'autre demi-anse, ledit implant comportant, de plus, un moyen de blocage et de réglage de la position de fermeture desdites extrémités libres courbées se chevauchant.

L'implant inter-épineux, selon l'invention, possède des moyens d'ancrage aux apophyses épineuses particulièrement avantageux. En effet, lorsque les demi-anses se rejoignent par leurs extrémités libres courbées dans une position dite de fermeture, elles enserrent, telles des anses, les apophyses épineuses. L'enserrage des apophyses épineuses par les moyens d'enserrage est assuré par un moyen de blocage. L'ancrage de l'implant selon l'invention aux apophyses épineuses ne nécessite pas de perforation de celles-ci et ne les fragilise donc pas.

Le moyen de blocage est également un moyen de réglage de la position des extrémités libres courbées l'une par rapport à l'autre lorsque celles-ci se chevauchent dans la position de fermeture. Ceci permet l'adaptation de l'implant selon la taille des apophyses épineuses qui peut varier d'un patient à l'autre.

En outre, l'implant inter-épineux selon l'invention peut être implanté dans le rachis cervical, le rachis dorsal ou le rachis lombaire, c'est-à-dire entre des vertèbres non soudées entre elles.

La voie d'implantation de l'implant inter-épineux selon l'invention est postérieure.

Selon un mode de réalisation de l'invention, le moyen de blocage desdites extrémités libres courbées se chevauchant en position de fermeture est composé de crantages de types à dents de loup que comportent les extrémités courbées des demi-anses de chaque moyen d'enserrage, lesdites dents du crantage d'une demi-anse étant orientées dans un sens inverse à celui des dents du crantage de l'autre demi-anse. Le réglage de la position de fermeture d'un moyen d'enserrage est réalisé par crantage.

Lorsque les extrémités libres courbées se chevauchent en position de fermeture, le moyen de blocage et de réglage permet ainsi d'éviter un mouvement longitudinal des extrémités libres courbées l'une par rapport à l'autre, les crantages des demi-anses d'un même moyen d'enserrage étant complémentaires l'une de l'autre.

Avantageusement, ledit moyen de blocage et de réglage comporte, de plus, une boucle qui est prévue pour entourer lesdites extrémités libres courbées se chevauchant dans ladite position de fermeture et qui comporte un moyen de guidage de l'extrémité libre courbée d'une demi-anse sous l'extrémité libre courbée d'une autre demi-anse, ledit moyen de guidage étant fixé sur l'extrémité libre courbée de la demi-anse qui chevauche l'extrémité libre courbée de l'autre demi-anse.

Cette boucle permet avantageusement d'éviter un mouvement latéral des extrémités libres courbées l'une par rapport à l'autre. Le moyen de guidage de la boucle, qui est fixé sur l'extrémité libre courbée de la demi-anse chevauchant l'autre demi-anse, permet le guidage de cette dernière sous l'extrémité courbée de la demi-anse chevauchante.

Selon un mode de réalisation de l'invention, l'extrémité libre courbée de la demi-anse qui est chevauchée par l'extrémité libre courbée de l'autre demi-anse dans ladite position de fermeture est biseautée de manière à percer un ligament reliant les apophyses épineuses de deux vertèbres adjacentes lorsque ledit implant est implanté entre lesdites vertèbres. Ce mode de réalisation de l'invention permet d'assurer davantage la stabilité de l'implant une fois implanté.

Selon un autre mode de réalisation de l'invention, chaque demi-anse comporte, de plus, au moins un crantage formé dans sa face interne qui est prévue pour être appliquée contre l'apophyse épineuse d'une vertèbre lorsque ledit implant est implanté entre lesdites vertèbres.

Selon une caractéristique de l'invention, l'extrémité libre courbée de chaque demi-anse présente une largeur inférieure à la largeur de son autre extrémité. Cette caractéristique permet un mouvement des extrémités libres l'une par rapport à l'autre dans un plan longitudinal pendant le réglage de la position de fermeture.

Selon un autre mode de réalisation de l'invention, ledit corps central comporte au moins un crantage formé sur la face externe d'au moins une desdites branches prévues pour être appliquées contre l'apophyse épineuse d'une vertèbre lorsque ledit implant est implanté entre lesdites vertèbres.

Selon un mode de réalisation particulier de l'invention, lesdits moyens d'enserrage dudit implant sont décalés l'un par rapport à l'autre le long desdites branches dudit corps central de manière à pouvoir implanter au moins deux implants tels que l'implant défini précédemment, entre plusieurs vertèbres adjacentes. Ce mode de réalisation est ainsi particulièrement adapté à l'implantation de plusieurs implants dans la colonne vertébrale et notamment lorsque deux implants doivent être ancrés à la même apophyse épineuse.

Selon ce même mode de réalisation de l'invention, ledit corps central comprend un moyen de logement des extrémités libres courbées se chevauchant en position de fermeture d'un autre implant inter-épineux tel que décrit précédemment, ledit moyen de logement étant un évidement aménagé d'une des branches du corps central. Ce mode de réalisation permet d'éviter que deux implants selon l'invention, implantés entre plusieurs vertèbres adjacentes et ancrés sur la même apophyse épineuse ne se gênent.

Selon une caractéristique de l'invention, ledit implant inter-épineux est monobloc. Cette caractéristique facilite l'implantation et réduit le temps de l'intervention chirurgicale.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
La Fig. 1 représente schématiquement un implant inter-épineux implanté dans le rachis d'un patient selon un mode de réalisation de l'invention, en vue de profil,
La Fig. 2 représente une vue oblique d'un implant selon l'invention et comporte un agrandissement d'un moyen de blocage d'un implant inter-épineux selon l'invention,
La Fig. 3 représente une vue de profil d'un moyen de blocage d'un implant inter-épineux selon l'invention et,
La Fig. 4 représente schématiquement l'implantation de plusieurs implants inter-épineux selon l'invention implantés dans le rachis d'un patient, en vue de profil.

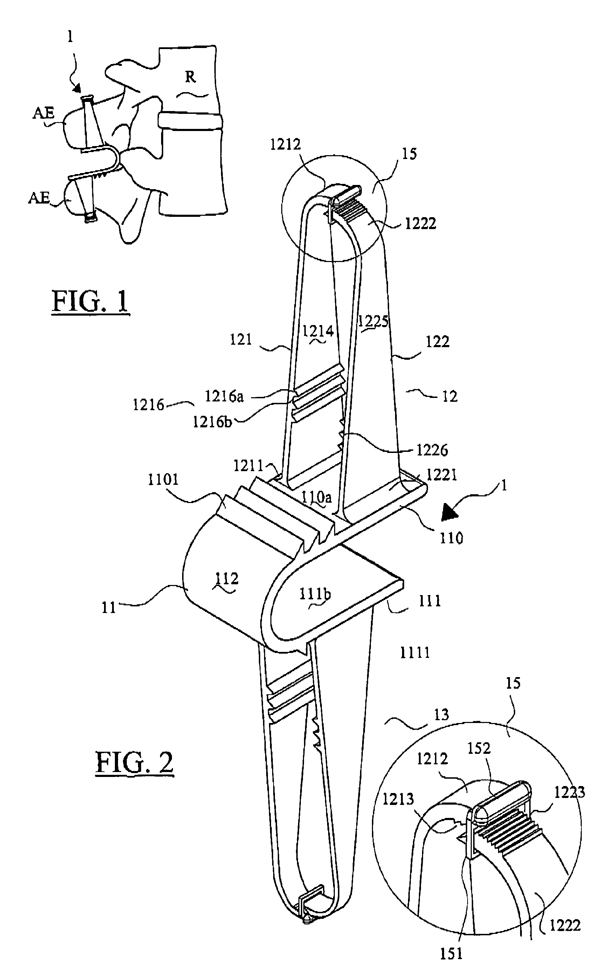

A la Fig. 1 est représenté un implant inter-épineux 1 implanté dans l'espace inter-épineux entre deux vertèbres adjacentes, une région dorsale et/ou lombaire du rachis R d'un patient.

L'implant 1 est composé d'un corps central 11 comprenant deux branches 111 et 110 reliées entre elles par un coude 112. Le corps central 11 présente ainsi une section sensiblement en forme de U.

A l'extrémité des faces extérieures des branches 110, 111 du corps central 11, font saillie deux moyens d'enserrage 12 et 13. Les moyens d'enserrage 12 et 13 sont perpendiculaires au plan du corps central 11 et forment ensemble des moyens d'ancrage de l'implant inter-épineux 1 aux apophyses épineuses AE de deux vertèbres, respectivement (Fig. 1).

L'implant inter-épineux 1 présente la caractéristique d'être monobloc. En effet, le corps central 11 et les moyens d'enserrage 12, 13 forment ensemble une unique pièce. Cela présente l'avantage de faciliter l'implantation par le chirurgien en limitant la durée.

L'implant inter-épineux 1 est réalisé entièrement en titane. Le titane présente la particularité d'être biocompatible, c'est-à-dire qu'il ne provoque normalement pas d'infection à l'intérieur du corps humain. Le titane présente également la caractéristique de ne pas dégrader les images obtenues par des procédés d'imagerie tels que la radiographie ou le scanner.

Le coude 112 permet d'absorber les contraintes mécaniques exercées sur les parties osseuses des vertèbres entre lesquelles l'implant est disposé.

L'implant intervertébral 1 procure au patient une mobilité dans le plan sagittal pour les deux vertèbres concernées. Il est intéressant de considérer que l'absence de mouvement dans le plan transversal (rotation gauche/droite) n'est généralement pas considérée comme rédhibitoire.

Les moyens d'enserrage 12 et 13 sont identiques et seul le moyen d'enserrage 12 sera décrit dans la suite de la présente description.

Le moyen d'enserrage 12 est composé de deux demi-anses 121, 122, parallèles entre elles. Les demi-anses 121, 122 présentent respectivement des extrémités 1211, 1221 liées au corps central 11, et des extrémités libres 1212, 1222.

L'extrémité libre 1212 de la demi-anse 121 est courbée en direction de l'extrémité libre 1222 de la demi-anse 122, et *vice versa*.

Les demi-anses 121, 122 sont d'une longueur telle que lorsque l'implant inter-épineux 1 est implanté dans le rachis R d'un patient, celles-ci se rejoignent dans une position de fermeture du moyen d'enserrage 12 autour de l'apophyse épineuse à laquelle il doit être ancré (Fig. 1).

Les extrémités courbées 1212, 1222 des demi-anses 121, 122, respectivement, comportent des crantages 1213, 1223. Le crantage 1213 est formé dans la face 1214 de la demi-anse 121, et présente une section de type en dent de scie comprenant une face inclinée 1213a sécante avec une face 1213b disposée perpendiculairement à la face 1214. Le crantage 1223 présente également une section de type en dent de scie comprenant une face inclinée 1223a sécante avec une face 1223b disposée perpendiculairement à la face 1215 de la demi-anse 122. Les dents du crantage 1213 sont orientées pour coopérer avec les dents du crantage 1223, lesquelles sont établies selon une orientation inverse.

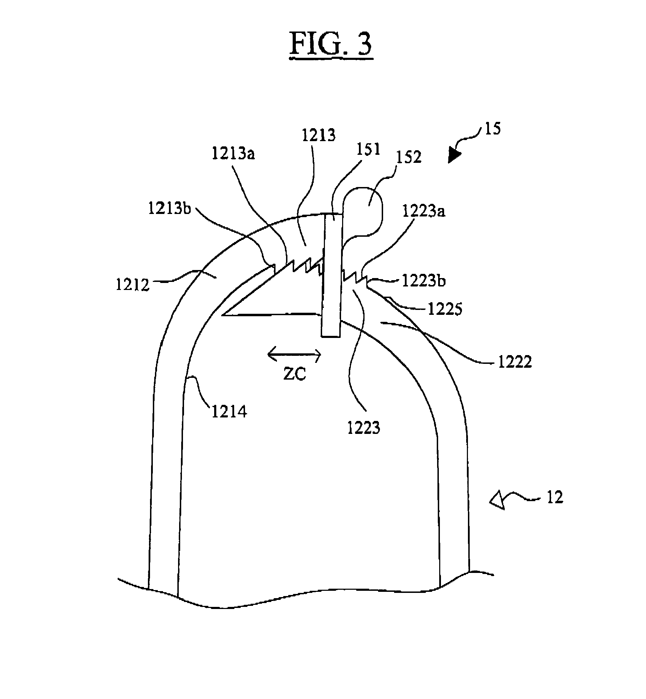

Dans la position de fermeture du moyen d'enserrage 12, illustrée à la Fig. 3, l'extrémité libre courbée 1212 de la demi-anse 121 chevauche l'extrémité libre courbée 1222 de la demi-anse 122, formant ainsi une zone de chevauchement ZC. Les crantages 1213, 1223 sont un moyen de blocage 15 de cette position de fermeture. En effet, dans cette position de fermeture, les crantages 1213, 1223 coopérant entre eux empêchent les extrémités courbées 1212 et 1222 de se déplacer l'une par rapport à l'autre dans un plan longitudinal.

Le moyen de blocage 15 est également un moyen de réglage de la position de l'extrémité libre courbée 1212 par rapport à l'extrémité libre courbée 1222 lorsqu'elles se chevauchent. En effet, la longueur de la zone de chevauchement ZC peut être réglée en fonction du crantage, c'est-à-dire du nombre de crans respectif des crantages 1213, 1223 engagés dans la zone de chevauchement.

Le moyen de blocage et de réglage 15 comprend, en outre, une boucle 151, dont la forme, dans ce mode de réalisation, est sensiblement rectangulaire (Fig. 2 et 3). La boucle 151 comporte un moyen de guidage 152 de l'extrémité libre courbée 1222 de la demi-anse 122 sous l'extrémité libre courbée 1212 de la demi-anse 121. Le moyen de guidage 152 est un butoir fixé sur l'extrémité libre courbée 1212 de la demi-anse 121 chevauchante. Dans la position de fermeture du moyen d'enserrage 12, les deux extrémités libres courbées 1212, 1222 se chevauchant sont à l'intérieur de la boucle 151, de telle sorte qu'un mouvement de translation de ces extrémités l'une par rapport à l'autre n'est pas permis. En conséquence, l'implant inter-épineux 1 n'est pas susceptible de se déloger de l'espace inter-épineux et demeure ancré de façon sûre aux apophyses épineuses AE.

Il est intéressant de noter que le moyen de blocage et de réglage 15 n'est pas une pièce ajoutée à l'implant inter-épineux 1, mais fait entièrement partie de l'implant.

Comme cela est visible sur la Fig. 2, les largeurs des extrémités libres courbées 1212, 1222 sont inférieures aux largeurs des extrémités 1211, 1221 en communication avec le corps central 11. Cette diminution des largeurs le long des demi-anses 121, 122 favorise le rapprochement des extrémités libres courbées 1211, 1221 l'une en direction de l'autre afin d'atteindre la position de fermeture du moyen d'enserrage 12.

L'extrémité libre courbée 1222 de la demi-anse 122, qui est chevauchée dans la position de fermeture du moyen d'enserrage 12, est biseautée de manière à pouvoir perforer un ligament (non représenté) reliant les deux apophyses épineuses AE sur lesquelles l'implant inter-épineux 1 est ancré. Cette caractéristique présente l'avantage de maintenir l'implant 1 dans l'espace inter-épineux.

Les demi-anses 121, 122 comportent, de plus, des crantages 1216, 1226 respectivement formés sur les faces 1214, 1224 qui sont prévues pour être placées contre les faces latérales des apophyses épineuses lorsque l'implant inter-épineux 1 est implanté entre deux vertèbres. Le crantage 1216 présente une section de type en dent de scie comprenant une face inclinée 1216a sécante avec une face 1216b disposée perpendiculairement à la face 1214. Le crantage 1226 est formé de façon identique au crantage 1216, sur la face 1224 de la demi-anse 122. Ces crantages favorisent l'ancrage de l'implant 1 aux apophyses épineuses des vertèbres.

De même, les branches 110, 111 du corps central 11 de l'implant 1 présentent sur leurs faces externes 110a, 111a (non visibles) des crantages 1101, 1111. Le crantage 1101 est destiné à être placé contre la paroi inférieure de l'apophyse épineuse d'une vertèbre alors que le crantage 1111 est destiné à être placé contre la paroi supérieure de l'apophyse épineuse de la vertèbre adjacente.

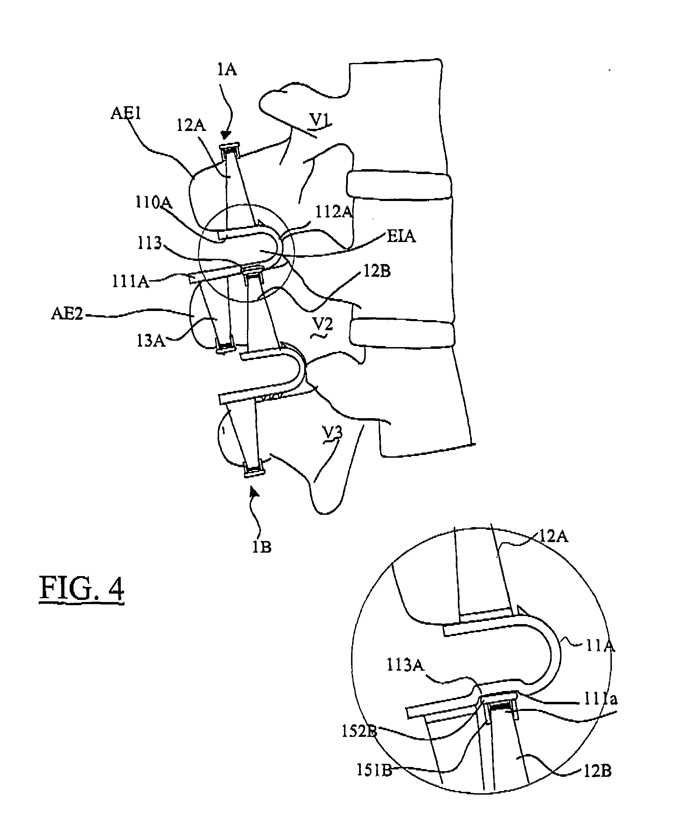

La Fig. 4 représente deux implants inter-épineux 1A, 1B, tels que l'implant inter-épineux 1, une fois implantés dans le rachis d'un patient, entre les vertèbres V1, V2 et V2, V3, respectivement. L'implant 1A est disposé de façon à ce que le coude 112A de l'implant 1A soit situé à l'intérieur de l'espace intersomatique EI 1. L'implant 1B est implanté de manière identique.

Dans un tel cas d'implantation multiple, les moyens d'enserrage 13A, 12B des implants 1A, 1 B, respectivement, sont ancrés sur la même apophyse épineuse AE2. Les moyens d'enserrage 12A, 13A de l'implant 1A sont décalés l'un par rapport à l'autre le long des branches 110A, 111A, respectivement, du corps central 11A, de manière à laisser un espace au moyen d'enserrage 12B autour de l'apophyse épineuse de la vertèbre V2. Dans un tel cas, il est judicieux que la longueur de la branche 110A de l'implant 1A soit inférieure à celle de la branche 111A.

Un moyen de logement 113A des extrémités libres courbées du moyen d'enserrage 13B de l'implant inter-épineux 1 B est prévu dans l'implant inter-épineux 1A afin d'éviter que le moyen d'enserrage 13B ne vienne buter contre la branche 111A de l'implant inter-épineux 1A. Le moyen de logement 113A est un évidement aménagé dans l'épaisseur de la face 111a de la branche 111A comme cela est visible sur la Fig. 4. L'évidement 113A est de forme complémentaire à celle des extrémités libres 1212B, 1222B se chevauchant en position de fermeture du moyen d'enserrage 12B de l'implant inter-épineux 1B, en tenant compte de la boucle 151B et du moyen de guidage 153B.

## Revendications

1. Implant intervertébral (1) destiné à être implanté entre deux vertèbres adjacentes, comprenant un corps central (11) sensiblement en forme de U comportant deux branches (110, 111), et deux moyens d'ancrage (12, 13) aux apophyses épineuses (AE) desdites vertèbres faisant saillie de la face extérieure (110a, 111 a) des deux branches (110, 111) dudit corps central (11), respectivement, **caractérisé en ce que** lesdits moyens d'ancrage (12, 13) sont des moyens d'enserrage desdites apophyses épineuses, chaque moyen d'enserrage (12) étant composé de deux demi-anses (121, 122) comportant chacune une extrémité libre courbée (1212) en direction de l'extrémité libre (1222) de l'autre demi-anse (122) et présentant chacune une longueur suffisante pour que, lorsque ledit implant (1) est implanté entre lesdites vertèbres, lesdites demi-anses (121, 122) se rejoignent dans une position de fermeture dudit moyen d'enserrage (12) autour de ladite apophyse épineuse (AE) dans laquelle l'extrémité libre courbée (1212) d'une demi-anse (12) chevauche l'extrémité libre courbée (1222) de l'autre demi-anse (122), ledit implant (1) comportant, de plus, un moyen de blocage et de réglage (15) de la position de fermeture desdites extrémités libres courbées se chevauchant (1212, 1222).

2. Implant intervertébral (1) selon la revendication 1, **caractérisé en ce que** ledit moyen de blocage et de réglage (15) de la position de fermeture desdites extrémités libres courbées (1212, 1222) se chevauchant est composé de crantages de type à dents de loup (1213, 1223) que comportent les extrémités libres courbées (1212, 1222) des demi-anses (121, 122), lesdites dents (1213a, 1213b) du crantage (1213) d'une demi-anse (121) étant orientées dans un sens inverse à celui des dents (1223a, 1223b) du crantage (1223) de l'autre demi-anse (122).

3. Implant intervertébral (1) selon l'une des revendications précédentes, **caractérisé en ce que** ledit moyen de blocage et de réglage (15) comporte, de plus, une boucle (151) qui est prévue pour entourer lesdites extrémités courbées (1212, 1222) se chevauchant dans ladite position de fermeture et qui comporte un moyen de guidage (152) de l'extrémité libre courbée (1222) sous l'extrémité libre courbée (1212), ledit moyen de guidage (152) étant fixé sur l'extrémité libre courbée (1212) de la demi-anse (121) qui chevauche l'extrémité libre courbée (1222) de l'autre demi-anse (122).

4. Implant intervertébral (1) selon l'une des revendications précédentes, **caractérisé en ce que** l'extrémité libre courbée (1222) de la demi-anse (122) qui est chevauchée par l'extrémité libre courbée (1212) de l'autre demi-anse (121) dans ladite position de fermeture est biseautée de manière à percer un ligament reliant les apophyses épineuses 5AE) de deux vertèbres adjacentes lorsque ledit implant (1) est implanté entre lesdites vertèbres.

5. Implant intervertébral (1) selon l'une des revendications précédentes, **caractérisé en ce que** chaque demi-anse (121) comporte, de plus, au moins un crantage (1216) formé dans sa face interne (1214) qui est prévue pour être appliquée contre l'apophyse épineuse (AE) d'une vertèbre lorsque ledit implant (1) est implanté entre lesdites vertèbres.

6. Implant intervertébral (1) selon l'une des revendications précédentes, **caractérisé en ce que** les extrémités libres courbées (1212, 1222) des demi-anses (121, 122) présentent des largeurs inférieures aux largeurs de leurs autres extrémités (1211, 1221).

7. Implant intervertébral (1) selon l'une des revendications précédentes, **caractérisé en ce que** ledit corps central (11) comporte au moins un crantage (1101) formé sur la face externe (110a) d'au moins une desdites branches (110) prévues pour être appliquées contre l'apophyse épineuse (AE) d'une vertèbre lorsque ledit implant (1) est implanté entre lesdites vertèbres.

8. Implant intervertébral (1) selon l'une des revendications précédentes, **caractérisé en ce que** lesdits moyens d'enserrage (12, 13) dudit implant (1) sont décalés l'un par rapport à l'autre le long desdites branches (110, 111) dudit corps central (11) de manière à pouvoir implanter au moins deux implants (1A, 1B) tels que l'implant (1) défini dans les revendications 1 à 7, entre plusieurs vertèbres adjacentes.

9. Implant intervertébral (1) selon la revendication 8, **caractérisé en ce que** ledit corps central (11) comprend un moyen de logement (113) des extrémités libres courbées (1212B, 1222B) se chevauchant en position de fermeture d'un autre implant inter-épineux (1B) tel que l'implant inter-épineux (1) défini dans les revendications 1 à 7, ledit moyen de logement (113) étant un évidement aménagé dans la surface d'une des branches (111) du corps central (11).

10. Implant intervertébral (1) selon l'une des revendications précédentes, **caractérisé en ce qu'**il est monobloc.
